# FASCICULE DE BREVET EUROPEEN

(11) **EP 2 726 087 B1**
(45) Date de publication et mention de la délivrance du brevet: **17.05.2017**
(21) Numéro de dépôt: 12729385.0
(22) Date de dépôt: 29.05.2012
(51) Int. Cl.: A61K 36/31, A61P 13/08, A61P 35/00, A23L 33/105, A61K 9/28

(54) **COMPOSITIONS POUR LE TRAITEMENT OU LA PRÉVENTION DU CANCER DE LA PROSTATE À BASE D'EXTRAIT DE GRAINES DE BROCOLI**
ZUSAMMENSETZUNGEN MIT BROKKOLISAMEN ZUR BEHANDLUNG ODER PRÄVENTION VON PROSTATAKREBS
COMPOSITIONS CONTAINING BROCCOLI SEEDS FOR TREATING OR PREVENTING PROSTATE CANCER

(30) Priorité: 01.07.2011 FR 1155926
(43) Date de publication de la demande: 07.05.2014
(73) Titulaire: SOJASUN TECHNOLOGIES, 35230 Noyal-sur-Vilaine (FR)
(72) Inventeur: EFSTATHIOU, Théo, F-35740 Pace (FR); PLU, Nicolas, F-35113 Domagne (FR)
(74) Mandataire: Vidon Brevets & Stratégie
(86) Numéro de dépôt international: PCT/EP2012/060069
(87) Numéro de publication internationale: WO 2013/004436

(56) Documents cités:
- WO-A1-2010/023162
- FR-A1- 2 888 235
- KRISTAL ALAN R ET AL: "Brassica vegetables and prostate cancer risk: a review of the epidemiological evidence", NUTRITION AND CANCER, LONDON, GB, vol. 42, no. 1, 1 janvier 2002 (2002-01-01), pages 1-9, XP008106063, ISSN: 0163-5581, DOI: 10.1207/S15327914NC421_1
- SINGH AJITA V ET AL: "Sulforaphane induces caspase-mediated apoptosis in cultured PC-3 human prostate cancer cells and retards growth of PC-3 xenografts in vivo", CARCINOGENESIS, OXFORD UNIVERSITY PRESS, GB, vol. 25, no. 1, 1 janvier 2004 (2004-01-01), pages 83-90, XP002591973, ISSN: 0143-3334, DOI: 10.1093/CARCIN/BGG178 [extrait le 2003-09-26]
- VERHOEVEN D T ET AL: "Epidemiological studies on brassica vegetables and cancer risk.", CANCER EPIDEMIOLOGY, BIOMARKERS & PREVENTION : A PUBLICATION OF THE AMERICAN ASSOCIATION FOR CANCER RESEARCH, COSPONSORED BY THE AMERICAN SOCIETY OF PREVENTIVE ONCOLOGY SEP 1996 LNKD- PUBMED:8877066, vol. 5, no. 9, septembre 1996 (1996-09), pages 733-748, XP002669900, ISSN: 1055-9965 cité dans la demande
- STEINMETZ K A ET AL: "Vegetables, fruit, and cancer prevention: a review.", JOURNAL OF THE AMERICAN DIETETIC ASSOCIATION OCT 1996 LNKD- PUBMED:8841165, vol. 96, no. 10, octobre 1996 (1996-10), pages 1027-1039, XP002669901, ISSN: 0002-8223 cité dans la demande
- ZHANG Y ET AL: "A major inducer of anticarcinogenic protective enzymes from broccoli: isolation and elucidation of structure", PROCEEDINGS OF THE NATIONAL ACADEMY OF SCIENCES OF USA, NATIONAL ACADEMY OF SCIENCE, WASHINGTON, DC; US, vol. 89, no. 6, 15 mars 1992 (1992-03-15), pages 2399-2403, XP000910544, ISSN: 0027-8424, DOI: 10.1073/PNAS.89.6.2399 cité dans la demande

## Description

### 1. Domaine de l'invention

Le domaine de l'invention est celui des compositions nutraceutiques ou pharmaceutiques pour le traitement ou la prévention du cancer de la prostate.

Plus précisément, l'invention concerne une composition pour le traitement ou la prévention du cancer de la prostate chez l'homme, à base d'extrait de brocoli.

### 2. Art antérieur

Le cancer de la prostate est la deuxième cause de mortalité en Europe et aux Etats-Unis et survient généralement chez l'individu de plus de 50 ans. Il existe plusieurs formes dont la plus commune et la plus sévère est l'adénocarcinome prostatique. D'abord très localisé, il peut s'étendre rapidement aux tissus avoisinants et former des métastases dans d'autres tissus (os, ganglions...).

Le traitement généralement appliqué est la résection chirurgicale complète de la prostate associée à un traitement comme la chimiothérapie, la curiethérapie ou la radiothérapie externe. Malgré tout, 30% à 50% des patients présentent une récidive biologique dans les 10 années suivant leur première prise en charge. La récidive biologique est la formation d'une tumeur maligne de même type histologique que la tumeur primitive, après un premier traitement à visée curative. Afin de surveiller cette récidive, le taux de Prostate Specifique Antigen (PSA) est régulièrement mesuré par dosage sérique du patient.

De plus, les seules options offertes au patient, en cas de récidive, sont :
- la surveillance simple suivie d'une déprivation androgénique en cas d'évolution rapide de la maladie, ou
- la déprivation androgénique précoce.

La déprivation androgénique consiste en une castration chimique ou chirurgicale du patient. Le cancer de la prostate étant une forme de cancer hormono-dépendant, cette solution radicale s'impose en dernier ressort. Malgré tout, son efficacité est limitée : des clones, indépendants de la stimulation hormonale, peuvent apparaître rapidement. À ce stade, le traitement se résume alors aux simples soins palliatifs.

Par ailleurs, les traitements associés à la résection chirurgicale provoquent de nombreux effets secondaires chez le patient. On peut citer à titre d'exemple les nausées, une importante fatigue, une modification de l'hémogramme, des troubles érectiles et urinaires, des troubles digestifs...

Il existe donc un besoin d'alternatives pour éviter ou du moins limiter la survenue de récidive chez des patients opérés, en limitant les effets secondaires et en réduisant la pénibilité du traitement pour le patient.

La consommation de crucifère est connue pour avoir un rôle positif dans le cancer de la prostate. L'actif en cause est le sulforaphane (4-(methylsulfinyl)butyl isothiocyanate), qui est un anti-oxydant puissant et connu pour avoir un rôle protecteur dans de nombreuses pathologies : l'asthme, l'inflammation des voies aériennes, les cancers du poumon, de l'estomac, du côlon, du rectum, de la prostate, du foie, du sein ... (Verhoeven and al, Epidemiological studies on brassica vegetables and cancer risk, Cancer Epidemiol Biomarkers Prev., 1996; Steinmetz and al. Vegetables, fruit and cancer prévention : a review, J. Am. Diet. Assoc.,1996 ; Zhang and al. A major inducer of anticarcinogenic protective enzymes from broccoli : isolation and elucidation of structure, Proc. Natl. Acad. Sci. USA, 1992). Le sulforaphane n'est pas présent dans les crucifères en tant que tel. Il est obtenu grâce à l'hydrolyse enzymatique de son précurseur, la glucoraphanine, par la myrosinase, une enzyme endogène des crucifères.

L'action chimiopréventive du sulforaphane serait due à ses propriétés antioxydantes, inhibitrices des enzymes de phase I (cytochrome P450) et activatrices des enzymes de phase Il (glutathion S transférase par exemple).

De plus, le sulforaphane possède en outre une activité pro-apoptotique puisqu'il bloque les cellules en phase G2/M, les empêchant de passer en phase mitotique, et activant les voies de l'apoptose. Il a été démontré que le sulforaphane est cytotoxique de manière dose-dépendante.

Des publications scientifiques ont rapporté les effets bénéfiques procurés par la consommation de crucifères du genre *Brassica,* tels que le chou et le brocoli, sur le risque de développement d'un cancer de la prostate (A.R. Kristal et al, Nutrition and Cancer, 2002; A. V.Singh et al., Carcinogenesis, 2004; K.A.Steinmetz et al., J. Am. Diet. Assoc., 1996). D'après d'autres travaux, cet effet protecteur serait dû à l'activation des enzymes de type Il par le sulforaphane (Y.Zhang et al, Proc.Natl.Acad.Sci. USA, 1992).

On connaît en outre la demande de brevet FR 2888235 A1 qui décrit un procédé d'extraction de sulforaphane à partir de broyat de cruciphères contenant de la glucoraphanine afin de produire un extrait aqueux de sulforaphane. On connaît également la demande WO2010/023162 A1 qui décrit un procédé de fabrication d'un extrait de glucosinolate sur colonne échangeuse d'ions.

Cependant, il n'a pas encore été porté à la connaissance de la Demanderesse que des compositions, ayant pour objectif de soigner ou prévenir la survenue du cancer de la prostate ou de sa récidive et utilisant uniquement des extraits de crucifère, aient été mises au point.

### 3. Objectifs de l'invention

L'invention a pour objectif de fournir une composition permettant de soigner ou de prévenir le cancer de la prostate ou sa récidive.

Un autre objectif de l'invention est de proposer, dans au moins un mode de réalisation, un procédé d'extraction d'un extrait non aqueux de graines de brocoli apte à entrer dans une telle composition permettant de traiter ou de prévenir le cancer de la prostate ou sa récidive.

### 4. Exposé de l'invention

Ces objectifs, ainsi que d'autres qui apparaîtront par la suite, sont atteints grâce à l'invention qui concerne une composition pour le traitement ou la prévention du cancer de la prostate ou de sa récidive qui comprend, à titre de principe actif, préférentiellement de seul principe actif, un extrait non aqueux de graines de brocoli non germées de la variété *Brassica Oleracea Italica,* ledit extrait étant riche en sulforaphane, comprenant au moins 10% en poids de sulforaphane et encapsulé par un composé choisi dans le groupe comprenant la gomme d'acacia, la maltodextrine et le mélange de ceux-ci, ladite composition se présentant sous la forme d'une gélule, d'un comprimé, d'une dragée ou d'un comprimé pelliculé, et étant administrée par voie orale une à trois fois par jour, à une dose comprise entre 2 et 200 mg/jour.

Ainsi, l'invention repose sur une approche innovante consistant à réaliser une composition permettant de prévenir ou guérir le cancer de la prostate ou sa récidive, grâce à l'utilisation d'un extrait non aqueux d'une variété particulière de brocoli qui s'est révélée très riche en sulforaphane. Les inventeurs ont en effet découvert que les graines non germées de la variété de brocoli *Brassica Oleracea Italica* sont particulièrement riches en glucoraphanine et qu'il convenait d'utiliser des extraits non aqueux de ces graines pour permettre l'obtention de compositions présentant une efficacité optimale.

Il est notoire que les graines germées sont riches en glucoraphanine. Le fait que de telles graines aient entamé leur processus de germination rend en pratique compliquée l'industrialisation de procédé d'extraction de principes actifs tels que la glucoraphanine à partir de ces mêmes graines. En effet, les plantules naissantes produisent des déchets qui colmatent les membranes de filtration utilisables dans le cadre de tels procédés. Il est alors nécessaire d'ajouter des étapes supplémentaires pour se débarrasser de ces déchets.

Les graines non germées de brocoli sont quant à elles connues pour contenir des quantités de principes actifs tels que la glucoraphanine beaucoup moins importantes que les graines germées et sont donc en pratique peu utilisée. Toutefois, les inventeurs ont découvert que les graines non germées d'une variété particulière de brocoli, à savoir *Brassica Oleracea Italica* présentent un taux en glucoraphanine suffisamment élevé pour les exploiter efficacement en vue de l'obtention d'extraits riches en ce principe actif.

Selon un mode de réalisation de l'invention, les compositions selon l'invention peuvent être à usage nutraceutique et être administrées une à trois fois par jour à une dose comprise entre 2 et jusqu'à 20 mg/jour. On entend par « composition nutraceutique », ou alicament, une composition à base d'aliment(s) possédant un effet positif et significatif sur la santé et la prévention de pathologies.

L'initiation de la carcinogenèse correspond à la mutation d'un gène dans une cellule, induite par une agression extérieure ou par une cause endogène. Parmi les agressions extérieures connues pour provoquer la carcinogenèse, on peut citer les rayonnements UV, la consommation de tabac et d'alcool.... Une des causes endogènes peut être le stress oxydatif résultant, par exemple, d'une inflammation chronique. Les deux mécanismes aboutissent à la formation de radicaux libres, provoquant des mutations de l'ADN. La prise régulière de doses faibles de sulforaphane permet donc à l'organisme d'intégrer et de potentialiser les bénéfices du sulforaphane dont les propriétés anti-oxydantes peuvent suppléer un déficit ponctuel du système de réparation de l'ADN (la glutathion S transférase par exemple). Grâce à la composition selon l'invention, la survenue d'une mutation carcinogène est évitée.

Par ailleurs, de telles compositions comportant de faibles doses de sulforaphane, autorise une consommation quotidienne comme nutraceutique, sans ressentir les effets cytotoxiques du sulforaphane. Elles peuvent donc bénéficier aux hommes adultes désireux de limiter les risques de développement d'un cancer de la prostate, en particulier lorsqu'il existe un terrain familial propice.

Selon un autre mode de réalisation de l'invention, les compositions selon l'invention sont à usage pharmaceutique et peuvent être administrées une à trois fois par jour à une dose comprise entre plus de 20 et 200 mg/jour.

De telles compositions peuvent être administrées quotidiennement à un patient ayant subi une résection chirurgicale de la prostate suite à un diagnostic de cancer de la prostate. Ces compositions peuvent être administrées à titre de traitement concomitant à une chimiothérapie, une radiothérapie ou une curiethérapie. Elles permettent donc de diminuer les doses administrées au patient. Cela aurait donc pour effet de diminuer l'impact négatif des médicaments sur le patient, notamment sur le foie. Cela permettrait en outre de diminuer la pénibilité du traitement pour le patient et son entourage.

De telles compositions peuvent également être administrées entre les cures de chimiothérapie, de curiethérapie ou de radiothérapie. Sans effet négatif sur l'organisme humain, elles sont bien supportées pour le patient tout en le protégeant efficacement et en ralentissant significativement le développement de tumeurs secondaires ou d'une récidive.

L'invention a encore pour objet un procédé d'obtention d'un extrait non aqueux de sulforaphane stabilisé par encapsulation pour la préparation d'une composition selon l'invention comprenant:
a. une étape de pressage mécanique à froid de graines de brocoli non germées de la variété *Brassica Oleracea Italica* conduisant à un tourteau partiellement délipidé,
b. une étape d'hydrolyse enzymatique dudit tourteau partiellement délipidé obtenu à l'étape a destinée à hydrolyser la glucoraphanine contenue dans lesdites graines en sulforaphane, et conduisant à l'obtention d'un hydrolysat,
c. une étape de purification dudit hydrolysat obtenu à l'étape b par addition d'acétone afin de réduire la teneur en lipides dudit hydrolysat obtenu à l'étape b, ainsi que sa teneur en acide érucique à une concentration inférieure à 5% en poids,
d. une étape de filtration dudit hydrolysat permettant la récupération d'un filtrat pauvre en lipide et en acide érussique,
e. une étape d'évaporation de ladite acétone contenue dans ledit filtrat obtenu à l'étape d pour récupérer une phase aqueuse,
f. une étape d'extraction du sulforaphane de ladite phase aqueuse obtenue à l'étape e par addition d'acétate d'éthyle pour récupérer une phase contenant ledit acétate d'éthyle et le sulforaphane,
g. une étape d'évaporation dudit acétate d'éthyle contenu dans ladite phase récupérée à l'étape f conduisant à l'obtention d'un extrait non aqueux riche en sulforaphane titrant à au moins 30% en poids de sulforaphane, (cet extrait non aqueux se présente sous la forme d'un produit visqueux),
h. une étape de séchage sur un support polysaccharidique choisi parmi le groupe comprenant la gomme d'acacia, la maltodextrine et leur mélange dudit extrait non aqueux obtenu à l'étape g conduisant à l'obtention d'une poudre,
i. une étape d'amélioration de la stabilité du sulforaphane présent dans ladite poudre obtenue à l'étape h par encapsulation dans une matrice polysaccharidique choisie parmi le groupe comprenant la gomme d'acacia, la maltodextrine et leur mélange, conduisant à une poudre encapsulée titrant à au moins 10% en poids de sulforaphane,
ledit procédé étant mené sans étape d'extraction aqueuse entre l'étape g et l'étape h.

Il est en effet impératif que l'extrait obtenu soit non aqueux. A ce sujet, on notera qu'il a déjà été proposé dans l'art antérieur un procédé d'extraction de sulforaphane à partir de graine de crucifères, notamment de brocoli conduisant à l'obtention d'un extrait aqueux. Ce procédé décrit dans le brevet FR2888235 conduit à l'obtention d'extraits aqueux qui se sont révélés inadaptés à la réalisation de compositions selon l'invention.

Grâce au procédé selon l'invention on peut réaliser des compositions à base de sulforaphane stabilisées et pouvant être conditionnées, commercialisées et stockées pour un usage pharmaceutique ou nutraceutique.

L'extraction par pressage mécanique permet d'éliminer une partie des lipides présents dans les graines de brocoli non germées. Le tourteau ainsi obtenu contient, entre autres éléments, la glucoraphanine et la myrosinase endogène du brocoli. L'addition d'eau permet d'activer la myrosinase et permet l'hydrolyse de la glucoraphanine en sulforaphane. L'hydrolysat ainsi obtenu contient encore des lipides qu'il est nécessaire d'éliminer, au moins en partie. Un de ces lipides est l'acide érucique ou acide 13-dococénoïque. L'acide érucique est un acide gras toxique pour l'animal. Il est donc nécessaire de réduire sa concentration à une teneur acceptable, à savoir inférieure à 5%. Cette réduction est obtenue en traitant l'hydrolysat par l'acétone. Ce solvant possède une grande affinité pour le sulforaphane. Il permet donc de poursuivre la délipidation du tourteau hydrolysé sans perdre le principe actif. De plus, ce solvant est autorisé en industrie alimentaire et pharmaceutique.

On obtient ainsi un hydrolysat pauvre en lipides et en acide érucique. L'hydrolysat est ensuite filtré puis séché pour éliminer tous les déchets solides et récupérer un filtrat débarrassé de l'acétone. Le sulforaphane est ensuite préférentiellement extrait grâce à l'addition d'acétate d'éthyle. Avantageusement, l'acétate d'éthyle est ajouté en rapport 1/1 par rapport à l'hydrolysat. Cette étape permet d'éliminer les molécules les plus polaires, comme les glucides et les polyphénols et certaines protéines. Le sulforaphane étant de polarité moyenne, l'utilisation de ce solvant permet de préserver la totalité du sulforaphane présent.

On obtient alors un extrait non aqueux qui comprend au moins 30% en poids de sulforaphane. Cet extrait non aqueux est ensuite séché sur une matrice de maltodextrine et de gomme arabique (E414). Le séchage de cette résine permet l'obtention d'une poudre. Les particules de cette poudre sont ensuite encapsulées par ces mêmes composants, c'est-à-dire la gomme arabique et la maltodextrine. Cette encapsulation permet de stabiliser les molécules de sulforaphane et d'obtenir une poudre titrée à 10% en sulforaphane. En effet, le sulforaphane est un composé extrêmement instable qui, à défaut d'une telle encapsulation, se dégrade très rapidement après sa formation.

Le procédé selon l'invention permet donc d'obtenir un extrait riche en sulforaphane, stabilisé et permettant son stockage dans des conditions normales de température et d'atmosphère pour la fabrication de compositions nutraceutiques ou pharmaceutiques ultérieurement.

L'encapsulation est réalisée grâce à toute méthode connue de l'homme de l'art permettant un enrobage par de la maltodextrine et de la gomme arabique (E414).

La poudre encapsulée obtenue grâce au procédé selon l'invention peut ensuite subir une étape supplémentaire de compression, selon toute méthode bien connue de l'homme de l'art, afin d'obtenir un comprimé.

Ce comprimé peut ensuite faire l'objet d'une dragéification, selon toute méthode bien connue de l'homme de l'art, pour former des comprimés dragéifiés ou dragées.

### 5. Description d'un mode de réalisation de l'invention

Le principe général de l'invention repose sur l'administration de doses de sulforaphane, de manière régulière, afin de soigner ou prévenir la récidive du cancer de la prostate.

Grâce au procédé selon l'invention décrit ci-dessus, on a obtenu un extrait non aqueux de sulforaphane stabilisé par enrobage se présentant sous la forme d'une poudre encapsulée titrant à 10% en poids de sulforaphane. Cette poudre a été comprimée puis dragéifiée pour donner une composition selon l'invention se présentant sous forme de dragée.

Dans le cadre du présent mode de réalisation, cette composition sous forme de dragée, présente la composition suivante :
Extrait non aqueux de graine de brocoli *Brassica oleracea* var. *italica* Diluant : Phosphate dicalcique, Cellulose microcristalline,
Anti-agglomérant : Carbonate de magnésium, Silice, Stéarate de magnésium ;
Agent d'enrobage : Gomme laque, Saccharose, Dioxyde de Titane, Polyvinyl pyrrolidone, Cire d'abeille, Cire de Carnauba, Talc ;
Colorant : Sucrose, Dioxyde de Titane (E171), Gomme d'Acacia (E414), Bleu brillant FCF (E133), Jaune de Quinoléine (E104), Sodium benzoate (E211), Indigotine (E132).

L'efficacité d'une telle composition contenant le sulforaphane encapsulé et se présentant sous forme de dragée a été testée.

Dans ce cadre seize patients ayant subi une résection chirurgicale de la prostate, suite à un diagnostic d'adénocarcinome de la prostate histologiquement prouvé (score de Gleason ≤ 7), ont été sélectionnés pour participer à l'étude. Ces patients ont tous présenté une récidive biologique définie par une augmentation du taux de PSA supérieur à 0,2 ng/ml et ont par conséquent été traités par radiothérapie externe complémentaire.

Ces patients ont reçu pendant 3 mois une dose quotidienne de 30 mg par jour *per os* soit trois dragées réparties dans la journée chacune contenant 10 mg en poids de sulforaphane.

Chaque patient a subit un examen clinique approfondi (dosages sanguins, hémogramme, pesée du patient ...).

Le taux de PSA de chaque patient a été dosé par prélèvement sanguin à t= 0, t= 14 jours, t= 30 jours, t= 3 mois. Des prélèvements à t=4 mois, t=5 mois, soit respectivement 1 mois et 2 mois après l'arrêt du traitement, ont également été réalisés pour observer l'évolution du taux de PSA après la fin du traitement.

L'efficacité du traitement a été évaluée grâce à la mesure du temps de doublement du taux de PSA. Il est défini comme le nombre de mois nécessaire pour que le taux de PSA d'un patient double. C'est un indicateur fiable de la virulence et de la rapidité de développement d'une métastase du cancer primitif de la prostate.

Pour 20% des patients, le taux de PSA a été fortement diminué au cours du traitement. Ce résultat suggère que la métastase produisant le PSA est en régression.

Pour 60% des patients, le temps de doublement du taux de PSA augmente pendant le traitement.

Pour 20% des patients restants, le temps de doublement du taux de PSA a continué d'augmenter malgré l'arrêt du traitement.

Parmi ces 80% de patients dont le temps de doublement du PSA a été augmenté, plus de la moitié présentait un facteur d'augmentation de 50% et un tiers d'entre eux présentait un facteur d'augmentation supérieur à 200%.

Il apparaît donc clairement qu'une composition pharmaceutique selon l'invention permet de freiner efficacement le temps de doublement du taux de PSA. Cette diminution signifie que la croissance de la tumeur à l'origine de la production de PSA est fortement ralentie. De plus, il est évidemment intéressant de pouvoir prolonger la vie du patient pour lui permettre une meilleure qualité de vie et pour pouvoir agir quand la tumeur est à un stade encore curable.

Au vu des résultats de cette étude, la composition selon l'invention permet donc de :
- diminuer le taux de PSA, traduisant une régression de la tumeur, ou
- ralentir considérablement l'évolution de la tumeur.

De plus, aucun de ces 16 patients n'a présenté de réaction allergique au traitement.

Dans d'autres modes de réalisation on pourra utiliser des compositions titrant à plus de 10 % en poids d'extrait de sulforaphane.

## Revendications

1. Composition pour son utilisation dans le traitement ou la prévention du cancer de la prostate ou de sa récidive comprenant à titre de principe actif un extrait non aqueux de graines non germées de brocoli de la variété *Brassica Oleracea Italica* riche ledit extrait comprenant au moins 10% en poids de sulforaphane et encapsulé par un composé choisi dans le groupe comprenant la gomme d'acacia, la maltodextrine et le mélange de ceux-ci, ladite composition se présentant sous la forme d'une gélule, d'un comprimé, d'une dragée ou d'un comprimé pelliculé, et étant administrée par voie orale une à trois fois par jour, à une dose comprise entre 2 et 200 mg/jour.

2. Composition pour son utilisation selon la revendication 1 **caractérisée en ce qu'**elle est à usage nutraceutique et **en ce qu'**elle est administrée une à trois fois par jour à une dose comprise entre 2 et jusqu'à 20 mg/jour.

3. Composition pour son utilisation selon la revendication 1 **caractérisée en ce qu'**elle est à usage pharmaceutique et **en ce qu'**elle est administrée une à trois fois par jour à une dose comprise entre plus de 20 et jusqu'à 200 mg/jour.

4. Procédé d'obtention d'un extrait non aqueux de sulforaphane stabilisé par encapsulation pour la préparation d'une composition selon l'une quelconque des revendications 1 à 3 comprenant:
a. une étape de pressage mécanique à froid de graines de brocoli non germées de la variété *Brassica Oleracea Italica* conduisant à un tourteau partiellement délipidé,
b. une étape d'hydrolyse enzymatique dudit tourteau partiellement délipidé obtenu à l'étape a destinée à hydrolyser la glucoraphanine contenue dans lesdites graines en sulforaphane, et conduisant à l'obtention d'un hydrolysat,
c. une étape de purification dudit hydrolysat obtenu à l'étape b par addition d'acétone afin de réduire la teneur en lipides dudit hydrolysat obtenu à l'étape b, ainsi que sa teneur en acide érucique à une concentration inférieure à 5% en poids,
d. une étape de filtration dudit hydrolysat permettant la récupération d'un filtrat pauvre en lipides et en acide érucique,
e. une étape d'évaporation de ladite acétone contenue dans ledit filtrat obtenu à l'étape d pour récupérer une phase aqueuse,
f. une étape d'extraction du sulforaphane de ladite phase aqueuse obtenue à l'étape e par addition d'acétate d'éthyle pour récupérer une phase contenant ledit acétate d'éthyle et le sulforaphane,
g. une étape d'évaporation dudit acétate d'éthyle contenu dans ladite phase récupérée à l'étape f conduisant à l'obtention d'un extrait non aqueux titrant à au moins 30% en poids de sulforaphane,
h. une étape de séchage sur un support polysaccharidique choisi parmi le groupe comprenant la gomme d'acacia, la maltodextrine et leur mélange dudit extrait huileux obtenu à l'étape g conduisant à l'obtention d'une poudre,
i. une étape d'amélioration de la stabilité du sulforaphane de ladite poudre obtenue à l'étape h par encapsulation dans une matrice polysaccharidique choisie parmi le groupe comprenant la gomme d'acacia, la maltodextrine et leur mélange, conduisant à une poudre encapsulée titrant à au moins 10% en poids de sulforaphane,
ledit procédé étant mené sans étape d'extraction aqueuse entre l'étape g et l'étape h.

## Patentansprüche

1. Zusammensetzung zur Verwendung in der Behandlung oder Vorbeugung von Prostatakrebs oder -rezidiv, umfassend als Wirkstoff einen reichhaltigen nichtwässrigen Extrakt von nichtgekeimten Samen von Brokkoli der Varietät *Brassica Oleracea Italica,* wobei der Extrakt mindestens 10 Gew.-% Sulforaphan umfasst und von einer Verbindung ausgewählt aus der Gruppe umfassend Gummi arabicum, Maltodextrin und die Mischung von diesen, verkapselt ist, wobei die Zusammensetzung in Form einer Gelatinekapsel, einer Tablette, eines Dragees oder einer Filmtablette vorliegt und auf dem oralen Weg ein- bis dreimal pro Tag in einer Dosis von zwischen 2 und 200 mg/Tag verabreicht wird.

2. Zusammensetzung zur Verwendung nach Anspruch 1, **dadurch gekennzeichnet, dass** sie der nutrazeutischen Verwendung dient und dass sie einbis dreimal pro Tag in einer Dosis von zwischen 2 und bis zu 20 mg/Tag verabreicht wird.

3. Zusammensetzung zur Verwendung nach Anspruch 1, **dadurch gekennzeichnet, dass** sie der pharmazeutischen Verwendung dient und dass sie ein- bis dreimal pro Tag in einer Dosis von zwischen mehr als 20 und bis zu 200 mg/Tag verabreicht wird.

4. Verfahren zur Gewinnung eines mittels Verkapselung stabilisierten nichtwässrigen Sulforaphanextrakts zur Herstellung einer Zusammensetzung nach einem der Ansprüche 1 bis 3, das Folgendes umfasst:
a. einen Schritt des mechanischen Kaltpressens von nichtgekeimten Brokkolisamen der Varietät *Brassica Oleracea Italica,* das zu einem teilentfetteten Presskuchen führt,
b. einen Schritt der enzymatischen Hydrolyse dieses im Schritt a erhaltenen teilentfetteten Presskuchens zum Hydrolysieren des in den Samen enthaltenen Glucoraphanins zu Sulforaphan, was zur Gewinnung eines Hydrolysats führt,
c. einen Schritt des Aufreinigens des in Schritt b erhaltenen Hydrolysats durch Versetzen mit Aceton, um den Lipidgehalt des in Schritt b erhaltenen Hydrolysats sowie seinen Erucasäuregehalt auf eine Konzentration von unter 5 Gew.-% zu vermindern,
d. einen Schritt der Filtration des Hydrolysats, wodurch sich ein lipid- und erucasäurearmes Filtrat gewinnen lässt,
e. einen Schritt des Abdampfens des in dem in Schritt d erhaltenen Filtrat enthaltenen Acetons, um eine wässrige Phase zu gewinnen,
f. einen Schritt des Extrahierens des Sulforaphans der in Schritt e erhaltenen wässrigen Phase durch Versetzen mit Essigester, um eine Phase, die den Essigester und das Sulforaphan enthält, zu gewinnen,
g. einen Schritt des Abdampfens des in der in Schritt f wiedergewonnenen Phase enthaltenen Essigesters, was zur Gewinnung eines nichtwässrigen Extrakts mit einem Gehalt von mindestens 30 Gew.-% Sulforaphan führt,
h. einen Schritt des Trocknens des in Schritt g erhaltenen öligen Extrakts auf einem Polysaccharidträger, ausgewählt aus der Gruppe umfassend Gummi arabicum, Maltodextrin und ihre Mischung, was zur Gewinnung eines Pulvers führt,
i. einen Schritt des Verbesserns der Stabilität des Sulforaphans des in Schritt h erhaltenen Pulvers durch Verkapseln in einer Polysaccharidmatrix, ausgewählt aus der Gruppe umfassend Gummi arabicum, Maltodextrin und ihre Mischung, was zu einem verkapselten Pulver mit einem Gehalt von mindestens 10 Gew.-% Sulforaphan führt,
wobei das Verfahren ohne einen Schritt des wässrigen Extrahierens zwischen Schritt g und Schritt h durchgeführt wird.

## Claims

1. Composition for the treatment or prevention of prostate cancer or its recurrence that comprises as an active principle a non-aqueous extract of non-sprouted broccoli seeds of the *Brassica Oleracea Italica* variety, said extract being rich in sulforaphane and being encapsulated by a compound selected from the group comprising acacia gum, maltodextrin and the mixture thereof, said composition taking the form of a capsule, a tablet, a coated pill or a film-coated tablet and being administered orally one to three times a day, in a dose of 2 to 200 mg/day.

2. Composition according to claim 1, **characterized in that** it is for nutraceutical use and **in that** it is administered one to three times a day in a dose ranging from to 2 to 20 mg/day.

3. Composition according to claim 1, **characterized in that** it is for pharmaceutical use and **in that** it is administered one to three times a day in a dose ranging from to 20 to 200 mg/day.

4. Method for obtaining a non-aqueous extract of sulforaphane stabilized by encapsulation for the preparation of a composition according to any one of the claims 1 to 3, comprising:
a. a step for mechanical cold pressing of the non-sprouted broccoli seeds of the *Brassica Oleracea Italica* variety leading to a partially delipidated cake;
b. a step of enzyme hydrolysis of said partially delipidated cake obtained at the step a for hydrolyzing the glucoraphanin contained in said seeds into sulforaphane, this step leading to the obtaining of a hydrolysate,
c. a step for purifying said hydrolysate obtained at the step b by addition of acetone to reduce the lipid content of said hydrolysate obtained at the step b, as well as its erucic acid content, to a concentration below 5% by weight;
d. a step for filtering said hydrolysate enabling the recovery of a filtrate with a low content of lipids and erucic acid,
e. a step for evaporating said acetone contained in said filtrate obtained at the step d for recovering an aqueous phase;
f. a step for extracting sulforaphane from said aqueous phase obtained at the step e by the addition of ethyl acetate to recover a phase containing said ethyl acetate and sulforaphane,
g. a step for evaporating said ethyl acetate contained in said phase recovered at the step f leading to the obtaining of a non-aqueous extract containing at least 30% by weight of sulforaphane;
h. a step for drying on a polysaccharide support selected from the group comprising acacia gum, maltodextrin and their mixture of said non-aqueous extract obtained at the step g leading to the obtaining of a powder,
i. a step for increasing the stability of the sulforaphane of said powder obtained at the step h by encapsulation in a polysaccharide matrix selected from the group comprising acacia gum, maltodextrin and the mixture thereof leading to an encapsulated powder containing at least 10% by weight of sulforaphane,
said method being performed without a step of aqueous extraction between the step g and the step h.
